**Europäisches Patentamt**

**European Patent Office**

**Office européen des brevets**

(19)

(11) Veröffentlichungsnummer: **0 238 928**
**B1**

(12) # EUROPÄISCHE PATENTSCHRIFT

(45) Veröffentlichungstag der Patentschrift:
13.06.90

(51) Int. Cl.⁵: **A61G 13/00, A61B 6/04**

(21) Anmeldenummer: 87103361.9

(22) Anmeldetag: 09.03.87

(54) **Patientenlagerungstisch.**

(30) Priorität: 20.03.86 DE 8607769 U

(43) Veröffentlichungstag der Anmeldung:
30.09.87 Patentblatt 87/40

(45) Bekanntmachung des Hinweises auf die Patenterteilung:
13.06.90 Patentblatt 90/24

(84) Benannte Vertragsstaaten:
DE FR

(56) Entgegenhaltungen:
DE-A- 1 196 815
DE-A- 2 204 573
DE-B- 1 158 663

(73) Patentinhaber: Siemens Aktiengesellschaft,
Wittelsbacherplatz 2, D-8000 München 2(DE)

(72) Erfinder: Schäfer, Willi, Dipl.-Ing. (FH),
Genglerstrasse 20, D-8520 Erlangen(DE)

## Beschreibung

Die Erfindung betrifft einen Patientenlagerungstisch mit einer einseitig an einem Sockel gehalterten Tischplatte.

Es ist ein Patientenlagerungstisch dieser Art bekannt, bei dem der Sockel an der Decke des Untersuchungsraumes aufgehängt ist. Die Tischplatte ragt dabei vom Sockel aus freitragend in den Raum, so daß ein guter Zugang zum Patienten möglich ist.

Der Erfindung liegt die Aufgabe zugrunde, einen Patientenlagerungstisch der eingangs genannten Art so auszubilden, daß ein leichtes Lösen der Tischplatte vom Sockel ohne Zuhilfenahme eines Werkzeuges möglich ist.

Diese Aufgabe ist erfindungsgemäß dadurch gelöst, daß die Tischplatte durch eine Steckverbindung mit dem Sockel verbunden ist, derart, daß sie durch Anheben an ihrem freien Ende und Bewegung in ihrer Längsrichtung vom Sockel weg von diesem lösbar ist. Bei dem erfindungsgemäßen Patientenlagerungstisch kann die Tischplatte durch einfaches Anheben vom Sockel gelöst und z.B. gegen eine andere Tischplatte ausgetauscht werden. Dabei besteht gleichzeitig ein Schutz gegen Auffahren von unten. Stößt nämlich die Tischplatte mit ihrem freien Ende unten an einem Hindernis an, so wird sie angehoben, ohne daß ein Schaden entsteht. Bei abgenommener Tischplatte ist der Tisch ohne verdeckte Flächen und damit gut zu reinigen.

Die Erfindung ist nachfolgend anhand eines in der Zeichnung dargestellten Ausführungsbeispieles näher erläutert.

Es zeigen:

Fig. 1 einen Patientenlagerungstisch nach der Erfindung, und

Fig. 2 eine Einzelheit des Patientenlagerungstisches gemäß Figur 1.

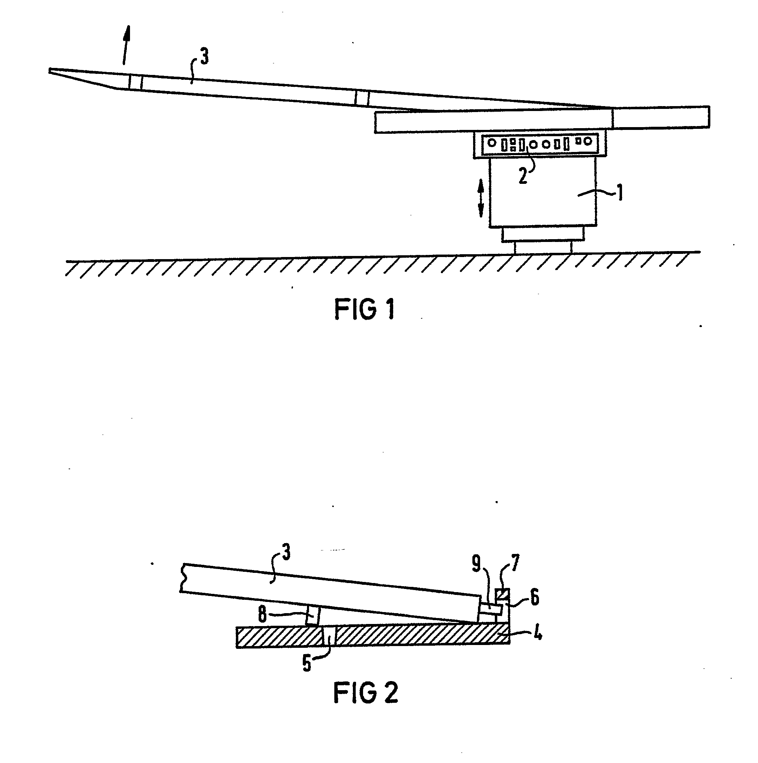

In der Figur 1 ist ein Sockel 1 eines Patientenlagerungstisches dargestellt, der aus teleskopartig in vertikaler Richtung ausfahrbaren Einzelteilen besteht, so daß der Patientenlagerungstisch höhenverstellbar ist. Die Steuerung des Tisches und eines zugeordneten Röntgenuntersuchungsgerätes erfolgt durch ein an der Seite des Sockels 1 angebrachtes Bedienkästchen 2. An der Oberseite des Sockels 1 ist eine Tischplatte 3 einseitig freitragend gehaltert.

Aus der Figur 1 geht hervor, daß die Tischplatte 3 leicht und ohne Werkzeug vom Sockel 1 gelöst werden kann, indem sie an ihrem freien Teil angehoben und dann in ihrer Längsrichtung in Richtung auf ihr freies Ende etwas verschoben wird. Sie ist hierzu durch eine Steckverbindung mit dem Sockel 1 verbunden.

Die Figur 2 diese Steckverbindung genauer. In der Figur 2 ist eine Platte 4 dargestellt, die auf der Oberseite des Sockels 1 befestigt ist. Die Platte 4 besitzt zwei Löcher 5, die sich in vertikaler Richtung erstrecken, und zwei horizontale Löcher 6 an einem Ansatz 7 an ihrem Ende. Die Tischplatte 3 weist zu den Löchern 5, 6 passende Zapfen 8, 9 auf. In der in der Figur 2 dargestellten Stellung der Tischplatte 3 ist diese an ihrem linken Teil angehoben und kann demgemäß von der Platte 4 leicht entfernt werden. Soll sie mit der Platte 4 verbunden werden, so wird sie aus der in der Figur 2 dargestellten Lage nach rechts verschoben, bis die Zapfen 9 ganz in den Löchern 6 stecken. Sie kann dann in ihrem linken Teil nach unten abgesenkt werden, wobei die Zapfen 8 in die Löcher 5 eingreifen.

Bei einem vorbestimmten Aufkippwinkel ist demgemäß die Tischplatte 3 wechselbar. Zum Lösen der Verbindung sind keinerlei Verriegelungen zu betätigen. Das Betätigen solcher Verriegelungen entfällt auch, wenn die Tischplatte 3 auf dem Sockel 1 gehaltert werden soll. Eine nicht richtig eingesetzte Tischplatte 3 ist klar erkennbar: Die vorderen Zapfen 8 rasten nicht in den Löchern 5 ein, so daß die Tischplatte 3 mit ihrem freien Ende sichtlich hochsteht.

## Patentansprüche

1. Patientenlagerungstisch mit einer einseitig an einem Sockel (1) gehalterten Tischplatte (3), dadurch gekennzeichnet, daß die Tischplatte (3) durch eine Steckverbindung (4 bis 9) mit dem Sockel (1) verbunden ist, derart, daß sie durch Anheben an ihrem freien Ende und Bewegung ihrer Längsrichtung vom Sockel (1) weg von diesem lösbar ist.

2. Patientenlagerungstisch nach Anspruch 1, dadurch gekennzeichnet, daß das sockelseitige Ende der Tischplatte (3) mit zwei nach unten und zwei vom Ende aus in Längsrichtung abstehenden Zapfen (8, 9) versehen ist, die in entsprechende Löcher (5, 6) des Sockeloberteiles (4) passen.

## Claims

1. Patient positioning table with a table top (3) mounted on one side on a base (1), characterised in that the table top (3) is connected by a plug connection (4 to 9) to the base (1), in such a way that the said table top can be detached from the base (1) by lifting it by its free end and moving it in its longitudinal direction away from the base.

2. Patient positioning table according to claim 1, characterised in that the end of the table top (3) is provided on the base side with two pins (8) which project downwards from the end in longitudinal direction and two pins (9) which project outwards from the end in longitudinal direction, which pins fit into corresponding holes (5, 6) in the upper part (4) of the base.

## Revendications

1. Table formant couchette pour patient comportant un plateau (3) monté unilatéralement sur un socle (1), caractérisée par le fait que le plateau (3) de la table est relié par une liaison à enfichage (4 à 9) au socle (1) de telle sorte qu'il peut être détaché du socle (1) par soulèvement au niveau de son extrémité libre et déplacement dans sa direction longitudinale.

2. Table formant couchette pour patient suivant la revendication 1, caractérisée par le fait que l'extrémité du plateau (3) de la table située du côté du socle, comporte deux tétons (8) dirigés vers le bas et deux tétons (9) qui font saillie dans la direction longitudinale à partir de l'extrémité et s'engagent dans des trous correspondants (5, 6) de la partie supérieure (4) du socle.

FIG 1

FIG 2